(19) Europäisches Patentamt
European Patent Office
Office européen des brevets

(11) Veröffentlichungsnummer : **0 116 318**
**B1**

(12) # EUROPÄISCHE PATENTSCHRIFT

(45) Veröffentlichungstag der Patentschrift : **02.03.88**

(51) Int. Cl.⁴ : **A 61 K 7/48**, C 07 D279/06

(21) Anmeldenummer : **84100530.9**

(22) Anmeldetag : **19.01.84**

(54) **Tetrahydro-1,3-thiazin-2,4-dione, ihre Verwendung sowie diese Verbindungen enthaltende Hautbehandlungsmittel.**

(30) Priorität : **12.02.83 DE 3304871**

(43) Veröffentlichungstag der Anmeldung : **22.08.84 Patentblatt 84/34**

(45) Bekanntmachung des Hinweises auf die Patenterteilung : **02.03.88 Patentblatt 88/09**

(84) Benannte Vertragsstaaten : **AT BE CH DE FR GB IT LI LU NL SE**

(56) Entgegenhaltungen :
**DE-A- 2 938 418**
**US-A- 4 352 929**
**CHEMICAL ABSTRACTS, Band 99, Nr. 11, 12. September 1983, Seite 564, Nr. 88131w, Columbus Ohio, USA W. HANEFELD: "Studies on 1,3-thiazines. 22. Oxidation of thiourethanes. 2. Oxidative desulfuration of 2-thixotetrahydro-1,3-thiazin-4-ones to tetrahydro-1,3-thiazine-2,4-diones with chromic acid anhydride"**

(73) Patentinhaber : **Wella Aktiengesellschaft**
**Berliner Allee 65**
**D-6100 Darmstadt (DE)**

(72) Erfinder : **Hanefeld, Wolfgang, Prof.Dr.**
**Ernst-Lemmer-Strasse 63**
**D-3550 Marburg/Lahn (DE)**
Erfinder : **Röthlisberger, Rudi, Dr.**
**Chemin des Cossettes 2**
**CH-1723 Marly (CH)**
Erfinder : **Noser, Friedrich, Dr.**
**La Halta**
**CH-1711 Bonnefontaine (CH)**

**EP 0 116 318 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Tetrahydro-1,3-thiazin-2,4-dione sowie Hautbehandlungsmittel mit einem Gehalt an diesen Verbindungen.

Die Haut bildet die Grenzschicht zwischen dem Organismus und seiner Umwelt. Die wichtigste Aufgabe der Haut besteht deshalb darin, das Körperinnere gegen exogene Einflüsse zu schützen. Unsere Haut steht täglich mit körperfremden, zum Teil körper- und speziell hautfeindlichen, Stoffen in Kontakt. Besonders häufiger Kontakt der ungeschützten Haut mit diesen Stoffen, der oft berufsbedingt ist (Friseure, Zahnärzte, Hausfrauen), führt früher oder später zu mehr oder weniger schweren Hautschäden. Zur Verhinderung oder zumindest zur Verminderung dieser Hautschäden wurde bisher prinzipiell auf zwei Arten eingegriffen : Durch protektiven Hautschutz sowie durch konservierende Hautpflege.

Der protektive Hautschutz besteht darin, daß man die Haut vor dem Kontakt mit dem hautfremden Mittel behandelt, um damit den direkten Kontakt zwischen der Hautoberfläche und den schädigenden Stoffen weitgehend auszuschließen. Die Präparate, die einen protektiven Hautschutz gewähren, üben ihre Wirkung chemisch-physikalisch aus, ohne in die Physiologie der Haut einzugreifen. Solche Präparate müssen insbesondere die folgenden Anforderungen erfüllen : Sie sollen undurchlässig und unlöslich gegenüber den meisten exogenen Noxen sein ; sie sollen eine gute Hautverträglichkeit aufweisen ; sie sollen leicht aufzutragen und auch wieder von der Hautoberfläche zu entfernen sein ; sie sollen die Griffigkeit der Hände und damit die Arbeitsfähigkeit nicht beeinträchtigen und sollen eine gewisse Erhaltungsdauer aufweisen. Der Nachteil von bekannten Präparaten dieser Art liegt eindeutig darin, daß sie nicht in der Lage sind, alle diese Ansprüche optimal zu erfüllen.

Bei der konservierenden Hautpflege geht es darum, die Haut beim Kontakt mit hautschädlichen Mitteln weniger anfällig zu machen. Die Hautschutzstoffe sind bereits in den Waschmitteln enthalten. Man unterscheidet ihrer Wirkung nach verschiedene Arten von Hautschutzmaßnahmen, und zwar solche, die durch Adsorption an der Hautoberfläche wirken, rückfettende Maßnahmen, azidifizierende Maßnahmen und entquellende Maßnahmen. Auch hier besteht der wesentliche Nachteil dieser Hautschutzmaßnahmen darin, daß sie nicht gegen alle Aggressionen der verschiedenen Umweltnoxen in gleicher Weise wirksam sind.

Die Verwendung von N-fluorbenzolsubstituierten, an $C_5$ und $C_6$ unsubstituierten Tetrahydro-1,3-thiazin-2,4-dionen mit immunsupressiven Eigenschaften zur Behandlung von Erkrankungen durch Überreaktion des Immunsystems ist in der US-A-4 352 929 beschrieben.

Nunmehr wurde gefunden, daß Hautbehandlungsmittel mit einem Gehalt an Tetrahydro-1,3-thiazin-2,4-dionen der allgemeinen Formel I

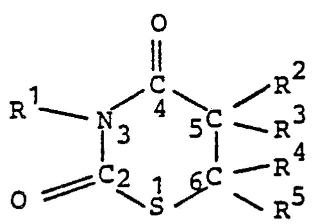

(I)

in der

$R^1$ einen der Reste H, Alkyl, Hydroxyalkyl, Carboxyalkyl, Halogenalkyl, Cyanoalkyl, Alkoxyalkyl, Phenyl, Phenylalkyl, in 4-Stellung Alkyl-, Halogen-, Nitro-, Alkoxy-, Phenyloxy- oder Cyano-substituiertes Phenyl oder Phenylalkyl, Pyridyl, Thiazolyl, Benzthiazolyl, Thienyl, Furyl, Pyrazolyl, Imidazolyl, Pyridazinyl, Thiadiazolyl, Pyrimidinyl und Thiazinyl darstellt,

$R^2$ und $R^3$ unabhängig voneinander H, Halogen, Alkyl, Carboxyl, Phenyl, Phenylalkyl, in 4-Stellung Alkyl-substituiertes Phenyl bedeuten,

$R^4$ einen der Reste H, Halogen, Alkyl, Halogenalkyl, Carboxyalkyl, Phenylalkyl, Phenyl, in 4-Stellung Alkyl-substituiertes Phenyl, 2-Furyl, substituiertes 2-Furyl ist und

$R^5$ die Bedeutung H, Halogen, Alkyl, Phenylalkyl, Phenyl oder in 4-Stellung Alkyl-substituiertes Phenyl hat (wobei « alkyl » jeweils für niedrigalkyl steht) unter der Voraussetzung, daß mindestens einer der Reste $R^2$, $R^3$, $R^4$ und $R^5$ nicht H ist, wenn $R^1$ Halogen-substituiertes Phenyl darstellt,

infolge ihrer neuartigen Wirksamkeit, alle Anforderungen, die an ein Hautschutzpräparat gestellt werden erfüllen.

Gegenstand der hier beschriebenen Erfindung sind daher Hautbehandlungsmittel, enthaltend physiologisch verträgliche Träger- und Zusatzstoffe, gekennzeichnet durch einen Gehalt an mindestens einer Verbindung der allgemeinen Formel I.

Die erfindungsgemäßen Hautbehandlungsmittel können in beliebigen, für Hautbehandlungsmittel geeigneten Zubereitungsformen, wie zum Beispiel als klare, gefärbte oder trübe Lösung, als Dispersion, Emulsion, in Form eines Schaumes oder aber als eine aus einem Aerosolbehälter mittels einer Pumpe oder durch ein Treibgas zu versprühende Zubereitung vorliegen. Bevorzugt liegen sie jedoch als Salbe,

Creme oder Gel vor. Als Beispiele für erfindungsgemäß in Betracht kommende Zubereitungen seien insbesondere kosmetische Hautbehandlungsmittel, wie Tagescremes, Nachtcremes, Nährcremes, Hautschutzcremes, Sonnenschutzcremes, Sonnenschutzsprays, Kälteschutzcremes, sowie weiterhin Lippenstifte, Hautmilch-Zubereitungen, Hautlotionen und Hautschutzgele, genannt.

Die Konzentration der Verbindungen der allgemeinen Formel I beträgt in den Hautbehandlungsmitteln insgesamt etwa 0,1 bis 5 Gew.%, vorzugsweise 0,5 bis 3 Gew.%. Hierbei können die Verbindungen der Formel I jeweils alleine oder im Gemisch miteinander in den Mitteln vorliegen.

Die Zusammensetzung der Hautbehandlungsmittel stellt eine Mischung der Verbindungen gemäß Formel I mit den für solche Zubereitungen üblichen physiologisch verträglichen Bestandteilen, wie Träger- und Zusatzstoffe, dar.

Übliche Träger- und Zusatzstoffe in Lösungen, Cremes, Emulsionen oder Gelen sind zum Beispiel Lösungsmittel wie Wasser, niedere aliphatische Alkohole, beispielsweise Ethanol, Propanol und Isopropanol, oder Glykole wie Glycerin und Propylenglykol, weiterhin Netzmittel oder Emulgatoren aus den Klassen der anionischen, kationischen, amphoteren oder nichtionogenen oberflächenaktiven Substanzen wie Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Fettsäureester, Stärke, Cellulosederivate, Vaseline, Stearin, Ceresin, Paraffinöl und Fettsäuren sowie außerdem Pflegestoffe wie Lanolin, Lanolinderivate, Cholesterin, Pantothensäure, Sorbit, Betain, Mandelöl, Avocadoöl, Bienenwachs und Walrat.

Weitere übliche Zusatzstoffe sind zum Beispiel kosmetische Harze, Farbstoffe, Parfümöle, Treibgase sowie Konservierungsstoffe, wie z. B. p-Hydroxybenzoesäure, Sorbinsäure, Salicylsäure, Formaldehyd und Hexachlorophen.

Zur Salzbildung können die Mittel Basen, wie z. B. Triethanolamin, enthalten.

Die Herstellung der Hautbehandlungsmittel erfolgt in der für derartige Präparate üblichen Weise, indem die als Wirkstoff dienenden Verbindungen der Formel I mit den für die Hautbehandlungsmittel als Trägerstoff dienenden Bestandteilen vermischt werden und danach mit den weiteren Bestandteilen der Mittel zum fertigen Endprodukt konfektioniert werden. Die als Bestandteil der hier beschriebenen Hautbehandlungsmittel enthaltenen Verbindungen der allgemeinen Formel I bewirken nach wiederholter epicutaner Behandlung eine Verdickung der äußersten Hautschicht, nämlich der toten Hornschicht, welche ja in erster Linie für den natürlichen Hautschutz verantwortlich ist. Durch diese Verdickung der Hornschicht wird die Haut gegen den Kontakt mit Umweltnoxen jeder Art widerstandsfähiger und bildet auf diese Art und Weise den optimalen Hautschutz. Da der Hautschutz, der durch diese Verbindungen gewährleistet wird, darin besteht, daß der natürliche Hautschutz verstärkt wird, weisen die erfindungsgemäßen Hautschutzmittel, welche Verbindungen nach der Formel I enthalten, die Nachteile, welche mit konventionellen Hautschutzmitteln beobachtet werden, nicht auf. Die erfindungsgemäßen Hautbehandlungsmittel können zeitlich unabhängig vom Kontakt mit der hautfremden Noxe auf die Hautoberfläche aufgetragen werden. Sie werden deshalb niemals einen Arbeitsvorgang stören, da sie sich zu diesem Zeitpunkt nicht mehr auf der Hautoberfläche befinden. Der Hautschutz kann auch nicht entfernt werden (z. B. durch Waschen), da er ja durch den neuen Zustand der Haut (Verdickung) erreicht wird. Gleichzeitig bewirken die Verbindungen der allgemeinen Formel I eine Verstärkung des natürlichen Sonnenschutzes. Dieser zusätzliche Sonnenschutz wird ebenfalls durch die Verdickung der Hornschicht, die nach der Behandlung mit dem erfindungsgemäßen Hautbehandlungsmittel erzielt wird, erreicht. Eine Verdickung der Hornschicht bewirkt nämlich eine erhöhte Absorption der Licht- bzw. Sonnenstrahlen. Dieser neuartige prophylaktische Sonnenschutz (Prä-sun) weist gegenüber der mit den konventionellen Sonnenschutzmitteln erzielbaren Wirkung eindeutig Vorteile auf. Die üblichen Sonnenschutzmittel werden auf die Hautoberfläche aufgetragen, und dabei ist ihre Absorptionskapazität, d. h. ihre Lichtschutzfähigkeit, von der aufgetragenen Schichtdicke abhängig. Diese Präparate können stören, indem sie z. B. zu stark fetten und dadurch Kleider verschmutzen. Sie werden z. B. durch Baden oder Duschen wieder abgespült und müssen deshalb ständig neu aufgetragen werden. Die erfindungsgemäßen Mittel können dagegen zeitlich unabhängig von der Exposition an der Sonne aufgetragen werden, indem sie, z. B. schon 3 bis 4 Wochen vor einem Sommer-Urlaub, wiederholt epicutan appliziert werden, so daß sie zur Urlaubszeit dann, dank der verdickten Hornschicht, schon einen langdauernden, nicht abwaschbaren Schutz vor Sonnenbrand und sonstigen chronischen Lichtschäden bieten.

In ähnlicher Weise sind die erfindungsgemäßen Mittel durch ihren Gehalt an den Tetrahydro-1,3-thiazin-2,4-dionen der Formel I in der Lage, an exponierten und empfindlichen Körperstellen, wie z. B. an Gesicht und Händen, die schlechten Witterungsverhältnissen besonders ausgesetzt sind, einen Schutz vor Kälte zu bieten. Die Mittel kommen daher insbesondere auch für die Anwendung durch Skiläufer und Hochgebirgssportler, beispielsweise als vorbeugender Schutz gegen extreme Kälte, in Betracht. Dank ihrer hornschichtverdickenden Wirkung sind die erfindungsgemäßen Hautbehandlungsmittel in der Lage, einen wirksamen Kälteschutz zu bieten, der auch in diesem Falle nicht die bei hautbelastenden und auf der Hautoberfläche verbleibenden Präparaten unangenehm empfundenen Nebenwirkungen aufweist, denn auch hier handelt es sich lediglich um eine Verstärkung des natürlichen Kälteschutzes.

Mit zunehmendem Alter wird die äußere Schicht der Haut, die sogenannte Oberhaut oder Epidermis, immer dünner. Die Verdünnung der Epidermis ist dafür verantwortlich, daß die Hautoberfläche mit dem Alter ihr typisches pergamentartiges Aussehen erhält und daß Talgdrüsen, Retentionszysten, Pigmentflecken sowie feine Blutgefäße sichtbarer werden und die typische Beschaffenheit einer sogenannten Altershaut prägen. Indem die erfindungsgemäßen Mittel in der Lage sind, nicht nur die Hornschicht, die ja nur einen Teil der Epidermis darstellt, sondern die gesamte Epidermis zu verdicken, stellen sie ein wirksames Mittel zur prophylaktischen Behandlung der Hautalterung dar.

Die erfindungsgemäßen Hautbehandlungsmittel werden zweckmäßig in der Weise angewendet, daß sie beginnend etwa 3 bis 4 Wochen vor dem Zeitpunkt, zu dem eine Verdickung der Hornhaut bzw. der Epidermis vorliegen soll, wiederholt und vorzugsweise 1 bis 2-mal täglich auf die entsprechenden Hautbereiche aufgetragen werden.

Die hautverdickende Wirkung der erfindungsgemäßen Verbindungen wurde an haarlosen Mäusen folgendermaßen nachgewiesen :

Die Wirkstoffe wurden, in geeigneten Grundlagen eingearbeitet, während zweieinhalb Wochen, täglich, außer Samstag und Sonntag, auf die eine Körperseite von haarlosen Mäusen (hr/hr) epicutan appliziert. Am Ende der Behandlungszeit wurden die Tiere getötet, auf beiden Körperseiten eine ca. 1 × 1,5 cm große Hautfläche entnommen und histologisch bearbeitet. Die Dicke der Oberhaut wurde an ca. 100 Stellen ausgemessen und die durchschnittliche Hautdicke ermittelt. Die Verdickung der Oberhaut ergab sich dann durch den Quotienten aus der durchschnittlichen Dicke der behandelten Oberhaut und der durchschnittlichen Dicke der unbehandelten Haut. Dieser Quotient wird als Verdickungsfaktor bezeichnet. Die erfindungsgemäßen Verbindungen ergaben Verdickungsfaktoren, die zwischen 1,3 und 2,2 lagen.

Eine Anzahl der Tetrahydro-1,3-thiazin-2,4-dione der allgemeinen Formel I ist bereits nach verschiedenen, bekannten Verfahren hergestellt worden. Die Verfahren zeichnen sich größtenteils durch nicht universelle Anwendbarkeit aus. So konnten aus N-monosubstituierten Thioncarbamidsäureestern durch Erhitzen mit β-Halogenpropionsäuren[1,2] oder β-Propiolacton[3] in Acetanhydrid nur in Position 5 und Position 6 der allgemeinen Formel I unsubstituierte Verbindungen erhalten werden.

Aus Thioharnstoff und substituierten 3-Brompropionsäuren[4] oder α-substituierten β-Propiolactonen[5] oder substituierten Acrylsäuren[6] wurden hingegen nur am Ringstickstoff der allgemeinen Formel I unsubstituierte Verbindungen erhalten. Aus Alkylaminen, Kohlenoxysulfid, Alkali und β-Propiolacton nach Ansäuren erhaltene 3-(Carbamoylthio)-propionsäuren waren zu Verbindungen der allgemeinen Formel I cyclisiert worden, bei denen $R^1$ nur Alkyl sein konnte und die Reste $R^2$ bis $R^5$ = H darstellten[7].

Auch die bisher universellste Methode zur Herstellung von Verbindungen der allgemeinen Formel I, nach der Verbindungen mit Alkyl- und Arylresten in Position 3 darstellbar sind, bestehend in der Cyclisierung von N-Alkyl- oder Aryl-substituierten 3-(Carbamoylthio)-propionsäuren[8], führt nur zu Verbindungen mit $R^2$ bis $R^5$ = H.

Ebenfalls wurden Verbindungen der allgemeinen Formel I mit Alkyl- oder Arylresten in Position 3 und den Substituenten $R^2$ bis $R^5$ = H durch Oxidation der entsprechenden Tetrahydro-2-thioxo-1,3-thiazin-4-one mit Kaliumdichromat in essigsaurer oder schwefelsaurer Lösung erhalten[9]. Als nachteilig bei letztgenannten Verfahren erwies sich, daß das Kaliumdichromat auch in der Hitze im Reaktionsmedium Essigsäure nicht ausreichend löslich ist. Setzt man zur Löslichkeitsverbesserung Wasser zu, werden die schlecht wasserlöslichen Tetrahydro-2-thioxo-1,3-thiazin-4-one wieder ausgefällt und dadurch teilweise der Oxidation entzogen, so daß die Umsetzung unvollständig bleibt und Substanzgemische resultieren.

Verbindungen der allgemeinen Formel II

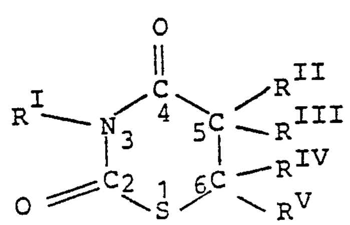

(II)

in der

$R^I$ einen der Reste H, Alkyl, Carboxyalkyl, Halogenalkyl, Alkoxyalkyl, Cycloalkyl, Arylalkyl, im Arylteil substituiertes Arylalkyl, Aryl, Alkyl-, Halogen-, Nitro-, Alkoxy-, Aryloxy-substituiertes Aryl, Pyridyl, Furyl,

[1] N.A. Langlet, Öfversigt af kongl. (Svenska) Vetenskaps-Akademiens Förhandlingar 1892, Seite 166

[2] E.V. Vladzimirskaya, Zh. Obshch. Khim. 31, Seite 1921 (1961) ; C.A. 55, 27328 (1961)

[3] W. Hanefeld, Justus Liebigs Ann. Chem. 1974, Seiten 2015-2018

[4] US-PS 2 585 064, K.W. Wheeler et al.

[5] GB-PS 1 007 587, G. Cignarella et al.

[6] K. Takemoto, H. Tahara, Y. Inaki, N. Ueda, Chem. Lett. 1972, Seite 767

[7] E. Campaigne, P.K. Nargund, J. Med. Chem. 7, (1964), Seite 132

[8] W. Hanefeld, Arch. Pharm. (Weinheim) 314, (1981), Seiten 315-328

[9] W. Hanefeld, Justus Liebigs Ann. Chem. 1974, Seiten 1789-1792

Pyrazolyl, Imidazolyl, Pyridazinyl, Pyrimidinyl darstellt und

$R^{II}$, $R^{III}$, $R^{IV}$ und $R^{V}$ unabhängig voneinander H, Alkyl, Cycloalkyl, Arylalkyl, Aryl und substituiertes Aryl bedeuten,

können hergestellt werden, indem man ein Tetrahydro-2-thioxo-1,3-thiazin-4-on der allgemeinen Formel

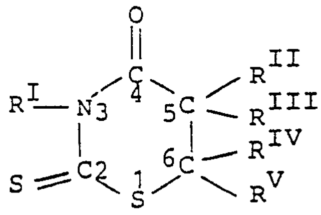

wobei die Substituenten $R^{I}$, $R^{II}$, $R^{III}$, $R^{IV}$ und $R^{V}$ die vorstehend genannte Bedeutung haben, in Essigsäure mit der dreifachen molaren Menge Chromsäureanhydrid ($CrO_3$) etwa eine Stunde zum Sieden erhitzt, das Reaktionsgemisch mit Wasser versetzt und nach dem Abkühlen das entstandene Tetrahydro-1,3-thiazin-2,4-dion der Formel II aus dem Reaktionsgemisch isoliert.

Die Darstellung der Verbindungen gemäß Formel II erfolgt bei diesem Verfahren in homogener Lösung, da sich $CrO_3$ in Essigsäure unter den verwendeten Konzentrationsbedingungen vollständig löst. Dadurch werden die oben zitierten, beim Arbeiten mit Kaliumdichromat als Oxidationsmittel auftretenden Schwierigkeiten vermieden. Dieses Verfahren ermöglicht weiterhin erstmalig die Darstellung von, am $C_5$ und/oder am $C_6$ der allgemeinen Formel II, substituierten Verbindungen bei gleichzeitiger Substitution am $N_3$.

Die vorliegende Anmeldung betrifft daher weiterhin neue, für die Verwendung in den erfindungsgemäßen Hautbehandlungsmitteln besonders geeignete, Tetrahydro-1,3-thiazin-2,4-dione der allgemeinen Formel III

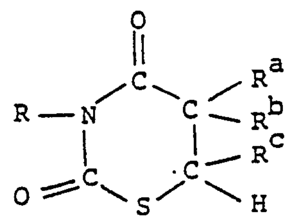

(III)

worin R die Bedeutung H, Alkyl, Phenyl, Phenylalkyl, in 4-Stellung Alkyl-, Halogen-, Nitro-, Alkoxy-, Phenyloxy- oder Cyano-substituiertes Phenyl oder Phenylalkyl hat, $R^a$ = H, Phenyl oder in 4-Stellung Alkyl-substituiertes Phenyl ist, $R^b$ = H, Phenyl oder in 4-Stellung Alkyl-substituiertes Phenyl ist und $R^c$ = H, Alkyl oder Phenyl darstellt (wobei « alkyl » jeweils für niedrigalkyl steht), unter der Vorraussetzung, daß $R^a$, $R^b$ und $R^c$ nur dann gleichzeitig H bedeuten, wenn R in 4-Stellung substituiertes Phenylalkyl ist und R nur dann H bedeutet, wenn $R^a$ = $R^b$ = Phenyl und $R^c$ = H darstellen.

Beispiele für erfindungsgemäße Verbindungen nach Formel III sind :

a) 3-(4-Chlorbenzyl)-tetrahydro-1,3-thiazin-2,4-dion
b) 3-(4-Chlorphenyl)-6-methyl-tetrahydro-1,3-thiazin-2,4-dion
c) 3-Benzyl-6-methyl-tetrahydro-1,3-thiazin-2,4-dion
d) 6-Methyl-3-(2-phenylethyl)-tetrahydro-1,3-thiazin-2,4-dion
e) 5,5-Diphenyl-tetrahydro-1,3-thiazin-2,4-dion
f) 3-Methyl-5,5-diphenyl-tetrahydro-1,3-thiazin-2,4-dion
g) 6-Phenyl-3-(4-methoxyphenyl)-tetrahydro-1,3-thiazin-2,4-dion
h) 6-Phenyl-3-(2-phenylethyl)-tetrahydro-1,3-thiazin-2,4-dion
i) 3-Ethyl-5,5-diphenyl-tetrahydro-1,3-thiazin-2,4-dion
j) 3-Methyl-5,5-di-(4-tolyl)-tetrahydro-1,3-thiazin-2,4-dion
k) 3-(4-Chlorphenyl)-5,5-diphenyl-tetrahydro-1,3-thiazin-2,4-dion
l) 3,5,5-Triphenyl-tetrahydro-1,3-thiazin-2,4-dion
m) 3-(4-Chlorbenzyl)-5,6-diphenyl-tetrahydro-1,3-thiazin-2,4-dion
n) 3-Benzyl-5,5-diphenyl-tetrahydro-1,3-thiazin-2,4-dion
o) 3-Benzyl-5,5-di-(4-tolyl)-tetrahydro-1,3-thiazin-2,4-dion

Die nachstehenden Beispiele sollen den Gegenstand der Erfindung näher erläutern.

## Beispiele für Hautbehandlungsmittel

Präparate für den Hautschutz

### Creme

| | |
|---|---|
| 2,0 g | 3-Benzyl-tetrahydro-1,3-thiazin-2,4-dion |
| 6,0 g | Gemisch aus 60 Gew.% Glycerinmonostearat und 40 Gew.% Glycerindistearat |
| 4,0 g | Polyoxyethylenglycerinmonostearat |
| 3,0 g | Cetylalkohol |
| 2,0 g | Paraffinöl, dickflüssig |
| 1,0 g | Lanolin |
| 0,3 g | Parfüm und Konservierungsmittel |
| 81,7 g | Wasser |
| 100,0 g | |

### Lotion

| | |
|---|---|
| 1,5 g | 3-Phenyl-tetrahydro-1,3-thiazin-2,4-dion |
| 0,5 g | Isopropyl-Lanolat |
| 3,0 g | Stearinsäure, dreifach gepreßt |
| 2,0 g | Glycerinmonostearat |
| 1,0 g | Triethanolamin |
| 0,3 g | Parfüm und Konservierungsmittel |
| 91,7 g | Wasser |
| 100,0 g | |

Prä-sun-Präparate (Sonnenschutzmittel)

### Milch

| | |
|---|---|
| 2,5 g | Tetrahydro-1,3-thiazin-2,4-dion |
| 3,0 g | Cetylphosphorsäure-diethanolaminsalz |
| 3,0 g | Stearinsäure, dreifach gepreßt |
| 5,0 g | Isopropylpalmitat |
| 5,0 g | Paraffinöl, dünnflüssig |
| 0,5 g | Parfüm und Konservierungsmittel |
| 81,0 g | Wasser |
| 100,0 g | |

### Körperlotion

| | |
|---|---|
| 2,0 g | 3-Benzyl-5,5-diphenyl-tetrahydro-1,3-thiazin-2,4-dion |
| 6,6 g | Propylenglycol-mono- und distearat, nicht selbstemulgierend (Monostearatgehalt 60 %) |
| 1,4 g | Triethanolamin |
| 1,0 g | Lanolin |
| 2,0 g | Isopropylmyristat |
| 2,0 g | 2-Octyldodecanol |
| 5,0 g | Avocadoöl |
| 2,6 g | Stearinsäure, dreifach gepreßt |
| 0,6 g | Ölsäure |
| 3,0 g | Sorbit |
| 0,5 g | Parfüm und Konservierungsmittel |
| 73,3 g | Wasser |
| 100,0 g | |

Präparate für den prophylaktischen Kälteschutz

Creme

| | |
|---|---|
| 5,0 g | 3-Benzyl-tetrahydro-1,3-thiazin-2,4-dion |
| 12,0 g | Glycerinmonostearat, selbstemulgierend |
| 10,0 g | Isocetylstearat |
| 0,3 g | Parfüm und Konservierungsmittel |
| 72,7 g | Wasser |
| 100,0 g | |

Emulsion

| | |
|---|---|
| 2,0 g | 5,5-Diphenyl-tetrahydro-1,3-thiazin-2,4-dion |
| 7,0 g | Glycerinmonostearat |
| 3,0 g | Cetylstearylalkohol |
| 10,0 g | Ölsäuredecylester |
| 10,0 g | Isopropylmyristat |
| 0,5 g | Parfüm und Konservierungsmittel |
| 67,5 g | Wasser |
| 100,0 g | |

Präparate zur prophylaktischen Behandlung der Altershaut

Nachtcreme

| | |
|---|---|
| 1,5 g | 3-(4-Chlorbenzyl)-tetrahydro-1,3-thiazin-2,4-dion |
| 22,0 g | Lanolinalkoholfraktionen |
| 5,0 g | Isopropylmyristat |
| 3,0 g | Ceresin (Paraffinum solidum) |
| 3,0 g | Lanolin |
| 5,0 g | Glycerin |
| 0,5 g | Parfüm und Konservierungsmittel |
| 60,0 g | Wasser |
| 100,0 g | |

Gesichts- und Halscreme

| | |
|---|---|
| 2,0 g | 3-Phenyl-tetrahydro-1,3-thiazin-2,4-dion |
| 3,0 g | Cetylphosphorsäure-diethanolaminsalz |
| 5,0 g | Stearinsäure, dreifach gepreßt |
| 15,3 g | Mandelöl |
| 10,0 g | Isopropylpalmitat |
| 5,0 g | Lanolin |
| 0,6 g | Parfüm und Konservierungsmittel |
| 59,1 g | Wasser |
| 100,0 g | |

Herstellungsbeispiele

Herstellung von 3-Methyl-5,5-diphenyl-tetrahydro-1,3-thiazin-2,4-dion (f) :

13,5 g (0,043 Mol) 3-Methyl-5,5-diphenyl-2-thioxo-tetrahydro-1,3-thiazin-4-on und 18,0 g (0,18 Mol) Chromsäureanhydrid werden in 100 g Essigsäure 1 Stunde zum Sieden erhitzt. Man versetzt mit Wasser bis zur beginnenden Trübung und kühlt ab. Die ausgefallenen Kristalle werden abgesaugt, mit Wasser gewaschen, getrocknet und aus Toluol/Petrolether umkristallisiert. Die physikalischen Daten und Analysenwerte sind der nachfolgenden Tabelle zu entnehmen.

Für die übrigen, in der nachfolgenden Tabelle aufgeführten erfindungsgemäßen Verbindungen der allgemeinen Formel III (Verbindungen a-e und g-o) erfolgt die Darstellung aus der entsprechenden 2-Thioxo-Verbindung in analoger Weise wie oben für die Verbindung (f) beschrieben. Als Lösungsmittel für die Umkristallisation kann dabei, je nach Substanz (siehe Tabelle), auch verdünnte Essigsäure, Chloroform/Petrolether oder Ethanol/$H_2O$ dienen. Die Größe der einzelnen Ansätze lag zwischen 1 und 50 mMol.

Tabelle : Erfindungsgemäße Tetrahydro-1,3-thiazin-2,4-dione der allgemeinen Formel III

| | R | $R^a$ | $R^b$ | $R^c$ | Schmelzpunkt °C (Umkristallisation aus) | Ausbeute % d. Theorie | Summenformel (Molmasse) | Analyse N berechnet / gefunden | S | Cl |
|---|---|---|---|---|---|---|---|---|---|---|
| a) | $4-Cl-C_6H_4-CH_2$ | H | H | H | 72-74,5 (verd. Essigsäure) | 50 | $C_{11}H_{10}ClNO_2S$ (255.7) | 5.48<br>5.32 | 12.54<br>12.46 | 13.87<br>13.87 |
| b) | $4-Cl-C_6H_4$ | H | H | $CH_3$ | 128-130 (verd. Essigsäure) | 26 | $C_{11}H_{10}ClNO_2S$ (255.7) | 5.48<br>5.55 | 12.54<br>12.79 | |
| c) | $C_6H_5-CH_2$ | H | H | $CH_3$ | 68-70 (verd. Essigsäure) | 27 | $C_{12}H_{13}NO_2S$ (235.3) | 5.95<br>5.94 | 13.63<br>13.34 | |
| d) | $C_6H_5-CH_2-CH_2$ | H | H | $CH_3$ | 113-115,5 (Toluol/Petrolether) | 64 | $C_{13}H_{15}NO_2S$ (249,3) | 5.62<br>5.38 | 12.86<br>12.83 | |
| e) | H | $C_6H_5$ | $C_6H_5$ | H | 181-183 (Chloroform/Petrolether) | 70 | $C_{16}H_{13}NO_2S$ (283,4) | 4.94<br>4.86 | 11.32<br>11.38 | |
| f) | $CH_3$ | $C_6H_5$ | $C_6H_5$ | H | 146 (Toluol/Petrolether) | 52 | $C_{17}H_{15}NO_2S$ (297.4) | 4.71<br>4.63 | 10.78<br>11.14 | |
| g) | $4-CH_3O-C_6H_4$ | H | H | $C_6H_5$ | 203-205 (Toluol/Ethanol) | 50 | $C_{17}H_{15}NO_3S$ (313,4) | 4.47<br>4.25 | 10.23<br>10.63 | |

Tabelle (Fortsetzung)

| | R | $R^a$ | $R^b$ | $R^c$ | Schmelzpunkt °C (Umkristallisation aus) | Ausbeute % d. Theorie | Summenformel (Molmasse) | Analyse N berechnet / gefunden | S | Cl |
|---|---|---|---|---|---|---|---|---|---|---|
| h) | $C_6H_5-CH_2-CH_2$ | H | H | $C_6H_5$ | 132-134<br>(Ethanol/$H_2O$) | 72 | $C_{18}H_{17}NO_2S$<br>(311,4) | 4.50<br>4.50 | 10.30<br>10.29 | |
| i) | $C_2H_5$ | $C_6H_5$ | $C_6H_5$ | H | 139-141<br>(Ether) | 67 | $C_{18}H_{17}NO_2S$<br>(311,4) | 4.50<br>4.52 | 10.30<br>10.25 | |
| j) | $CH_3$ | $4-CH_3-C_6H_4$ | $4-CH_3-C_6H_4$ | H | 175-176<br>(verd. Essigsäure) | 32 | $C_{19}H_{19}NO_2S$<br>(325,4) | 4.30<br>4.10 | 9.85<br>10.02 | |
| k) | $4-Cl-C_6H_4$ | $C_6H_5$ | $C_6H_5$ | H | 171-174<br>(verd. Essigsäure) | 74 | $C_{22}H_{16}ClNO_2S$<br>(393,9) | 3.56<br>3.54 | 8.14<br>7.82 | |
| l) | $C_6H_5$ | $C_6H_5$ | $C_6H_5$ | H | 167-169<br>(Ethanol/Petrolether) | 68 | $C_{22}H_{17}NO_2S$<br>(359,5) | 3.90<br>3.88 | 8.92<br>8.96 | |
| m) | $4-Cl-C_6H_4-CH_2$ | H | $C_6H_5$ | $C_6H_5$ | 149-150,5<br>(Toluol/Petrolether) | 70 | $C_{23}H_{18}ClNO_2S$<br>(407,9) | 3.43<br>3.39 | 7.86<br>7.91 | 8.69<br>8.40 |
| n) | $C_6H_5-CH_2$ | $C_6H_5$ | $C_6H_5$ | H | 181-182<br>(Toluol/Petrolether) | 64 | $C_{23}H_{19}NO_2S$<br>(373,5) | 3.75<br>3.50 | 8.59<br>8.53 | |

Tabelle (Fortsetzung)

| | R | $R^a$ | $R^b$ | $R^c$ | Schmelzpunkt °C (Umkristallisation aus) | Ausbeute % d. Theorie | Summenformel (Molmasse) | Analyse N berechnet gefunden | S | Cl |
|---|---|---|---|---|---|---|---|---|---|---|
| o) | $C_6H_5-CH_2$ | $4-CH_3-C_6H_4$ | $4-CH_3-C_6H_4$ | H | 177-180 (verd. Essigsäure) | 60 | $C_{25}H_{23}NO_2S$ (401,5) | 3.49<br>3.20 | 7.99<br>7.99 | |

Im Infrarotspektrum zeigen die erfindungsgemäßen Verbindungen eine Carbamoyl-C = O-Schwingung des $C_2$ bei 1630-1670 $cm^{-1}$ und eine Lactam-C = O-Schwingung des $C_4$ bei 1700-1720 $cm^{-1}$. Die Verbindung (e) zeigt zusätzlich eine NH-Bande bei 3320 $cm^{-1}$.

**Patentansprüche**

1. Hautbehandlungsmittel, enthaltend physiologisch verträgliche Träger- und Zusatzstoffe, gekennzeichnet durch einen Gehalt an mindestens einem Tetrahydro-1,3-thiazin-2,4-dion der allgemeinen Formel I

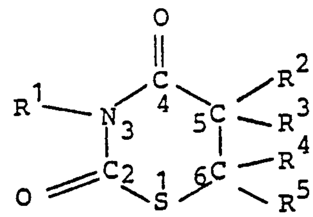

(I)

in der

$R^1$ einen der Reste H, Alkyl, Hydroxyalkyl, Carboxyalkyl, Halogenalkyl, Cyanoalkyl, Alkoxyalkyl, Phenyl, Phenylalkyl, in 4-Stellung Alkyl-, Halogen-, Nitro-, Alkoxy-, Phenyloxy- oder Cyano-substituiertes Phenyl oder Phenylalkyl, Pyridyl, Thiazolyl, Benzthiazolyl, Thienyl, Furyl, Pyrazolyl, Imidazolyl, Pyridazinyl, Thiadiazolyl, Pyrimidinyl und Thiazinyl darstellt,

$R^2$ und $R^3$ unabhängig voneinander H, Halogen, Alkyl, Carboxyl, Phenyl, Phenylalkyl, in 4-Stellung Alkyl-substituiertes Phenyl bedeuten,

$R^4$ einen der Reste H, Halogen, Alkyl, Halogenalkyl, Carboxyalkyl, Phenylalkyl, Phenyl, in 4-Stellung Alkyl-substituiertes Phenyl, 2-Furyl, substituiertes 2-Furyl ist und

$R^5$ die Bedeutung H, Halogen, Alkyl, Phenylalkyl, Phenyl oder in 4-Stellung Alkyl-substituiertes Phenyl hat (wobei « alkyl » jeweils für niedrigalkyl steht) unter der Voraussetzung, daß mindestens einer der Reste $R^2$, $R^3$, $R^4$ und $R^5$ nicht H ist, wenn $R^1$ in 4-Stellung Halogen-substituiertes Phenyl darstellt.

2. Mittel nach Anspruch 1, dadurch gekennzeichnet, daß die Verbindungen der Formel I in einer Menge von insgesamt 0,1 bis 5 Gew.%, vorzugsweise 0,5 bis 3 Gew.%, enthalten sind.

3. Hautbehandlungsmittel nach Anspruch 1 und 2, dadurch gekennzeichnet, daß es ein Hautschutzmittel ist.

4. Hautbehandlungsmittel nach Anspruch 1 und 2, dadurch gekennzeichnet, daß es ein prophylaktisches Sonnenschutzmittel (Prä-Sun Präparat) ist.

5. Hautbehandlungsmittel nach Anspruch 1 und 2, dadurch gekennzeichnet, daß es ein prophylaktisches Kälteschutzmittel ist.

6. Hautbehandlungsmittel nach Anspruch 1 und 2, dadurch gekennzeichnet, daß es ein prophylaktisches Mittel gegen die Hautalterung ist.

7. Mittel nach den Ansprüchen 1 bis 6, dadurch gekennzeichnet, daß es in Form einer Creme vorliegt.

8. Verwendung von Verbindungen der allgemeinen Formel I in Hautbehandlungsmitteln.

9. Verbindungen der allgemeinen Formel III

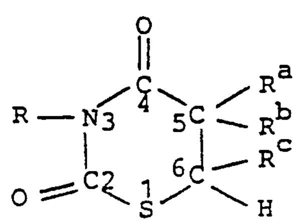

(III)

worin R die Bedeutung H, Alkyl, Phenyl, Phenylalkyl, in 4-Stellung Alkyl-, Halogen-, Nitro-, Alkoxy-, Phenyloxy- oder Cyano-substituiertes Phenyl oder Phenylalkyl hat, $R^a$ = H, Phenyl oder in 4-Stellung Alkyl-substituiertes Phenyl ist, $R^b$ = H, Phenyl oder in 4-Stellung Alkyl-substituiertes Phenyl ist und $R^c$ = H, Alkyl oder Phenyl darstellt (wobei « alkyl » jeweils für niedrigalkyl steht), unter der Vorraussetzung, daß $R^a$, $R^b$ und $R^c$ nur dann gleichzeitig H bedeuten, wenn R in 4-Stellung substituiertes Phenylalkyl ist und R nur dann H bedeutet, wenn $R^a$ = $R^b$ = Phenyl und $R^c$ = H darstellen.

10. Verwendung von Verbindungen der allgemeinen Formel III in Hautbehandlungsmitteln.

11. 3-(4-Chlorbenzyl)-tetrahydro-1,3-thiazin-2,4-dion.

12. 3-(4-Chlorphenyl)-6-methyl-tetrahydro-1,3-thiazin-2,4-dion.

13. 3-Benzyl-6-methyl-tetrahydro-1,3-thiazin-2,4-dion.

14. 6-Methyl-3-(2-phenylethyl)-tetrahydro-1,3-thiazin-2,4-dion.

15. 5,5-Diphenyl-tetrahydro-1,3-thiazin-2,4-dion.

16. 3-Methyl-5,5-diphenyl-tetrahydro-1,3-thiazin-2,4-dion.

17. 6-Phenyl-3-(4-methoxyphenyl)-tetrahydro-1,3-thiazin-2,4-dion.
18. 6-Phenyl-3-(2-phenylethyl)-tetrahydro-1,3-thiazin-2,4-dion.
19. 3-Ethyl-5,5-diphenyl-tetrahydro-1,3-thiazin-2,4-dion.
20. 3-Methyl-5,5-di-(4-tolyl)-tetrahydro-1,3-thiazin-2,4-dion.
21. 3-(4-Chlorphenyl)-5,5-diphenyl-tetrahydro-1,3-thiazin-2,4-dion.
22. 3,5,5-Triphenyl-tetrahydro-1,3-thiazin-2,4-dion.
23. 3-(4-Chlorbenzyl)-5,6-diphenyl-tetrahydro-1,3-thiazin-2,4-dion.
24. 3-Benzyl-5,5-diphenyl-tetrahydro-1,3-thiazin-2,4-dion.
25. 3-Benzyl-5,5-di-(4-tolyl)-tetrahydro-1,3-thiazin-2,4-dion.

## Claims

1. Skin treatment preparation containing physiological compatible carrier substances and additives, characterised in that it has a content of at least one tetrahydro-1,3-thiazine-2,4-dion of general formula I

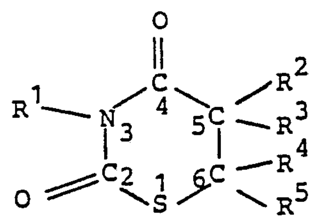

(I)

in which

$R^1$ represents one of the groups H, alkyl, hydroxyalkyl, carboxyalkyl, halogenalkyl, cyanoalkyl, alkoxyalkyl, phenyl, phenylalkyl, in 4th position alkyl-, halogen-, nitro-, alkoxy-, phenyloxy- or cyano-substituted phenyl or phenylalkyl, pyridyl, thiazolyl, benzthiazolyl, thienyl, furyl, pyrazolyl, imidazolyl, pyridazinyl, thiadiazolyl, pyrimidinyl and thiazinyl,

$R^2$ and $R^3$ represent independently of each other H, halogen, alkyl, carboxyl, phenyl, phenylalkyl and in 4th position alkyl-substituted phenyl,

$R^4$ constitutes one of the groups H, halogen, alkyl, halogenalkyl, carboxyalkyl, phenylalkyl, phenyl, in 4th position alkyl-substituted phenyl, 2-furyl and substituted 2-furyl and

$R^5$ represents H, halogen, alkyl, phenylalkyl, phenyl or in 4th position alkyl-substituted phenyl (whereby « alkyl » represents in each case low alkyl) on condition that at least one of the groups $R^2$, $R^3$, $R^4$ and $R^5$ is not H, if $R^1$ represents in 4th position halogen-substituted phenyl.

2. Preparation according to claim 1, characterised in that the compounds of formula I are contained in a quantity totalling 0.1 to 5 % by weight and for preference 0.5 to 3 % by weight.

3. Skin treatment preparation according to claims 1 and 2, characterised in that it represents a skin protection agent.

4. Skin treatment preparation according to claims 1 and 2, characterised in that it constitutes a prophylactic sun protection preparation (Pre-Sun preparation).

5. Skin treatment preparation according to claims 1 and 2, characterised in that it is a prophylactic preparation giving protection against the cold.

6. Skin treatment preparation according to claims 1 and 2, characterised in that it is a prophylactic preparation to prevent skin aging.

7. Preparation according to claims 1 to 6 characterised in that it has the form of a cream.

8. Use of compounds of general formula I in skin treatment preparations.

9. Compounds of the general formula III

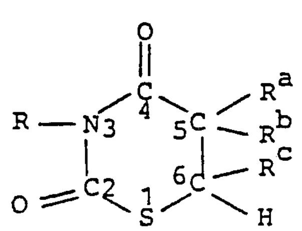

(III)

in which R represents H, alkyl, phenyl, phenylalkyl, in 4th position alkyl-, halogen-, nitro-, alkoxy-, phenyloxy- or cyano- substituted phenyl or phenyl alkyl, $R^a$ represents H, phenyl or in 4th position alkyl-substituted phenyl, $R^b$ represents H, phenyl or in 4th position alkyl-substituted phenyl and $R^c$ represents H, alkyl or phenyl (whereby « alkyl » in each case represents lower alkyl) on condition that $R^a$, $R^b$ and $R^c$ only represent H simultaneously, when R represents in 4th position substituted phenylalkyl and R only

represents H, when $R^a = R^b$ = represents phenyl and $R^c$ = H.

10. Use of compounds of the general formula III in skin treatment preparations.
11. 3-(4-Chlorobenzyl)-tetrahydro-1, 3-thiazine-2, 4-dion.
12. 3-(4-Chlorophenyl)-6-methyl-tetrahydro-1, 3-thiazine-2,4-dion.
13. 3-Benzyl-6-methyl-tetrahydro-1, 3-thiazine-2, 4-dion.
14. 6-Methyl-3-(2-phenylethyl)-tetrahydro-1, 3-thiazine-2,4-dion.
15. 5,5-Diphenyl-tetrahydro-1, 3-thiazine-2,4-dion.
16. 3-Methyl-5,5-diphenyl-tetrahydro-1,3-thiazine-2,4-dion.
17. 6-Phenyl-3-(4-methoxyphenyl)-tetrahydro-1,3-thiazine-2,4-dion.
18. 6-Phenyl-3-(2-phenylethyl)-tetrahydro-1,3-thiazine-2,4-dion.
19. 3-Ethyl-5,5-diphenyl-tetrahydro-1,3-thiazine-2,4-dion.
20. 3-Methyl-5,5-di(4-tolyl)-tetrahydro-1,3-thiazine-2,4-dion.
21. 3-(4-Chlorophenyl)-5,5-diphenyl-tetrahydro-1,3-thiazine-2,4-dion.
22. 3,5,5-Triphenyl-tetrahydro-1,3-thiazine-2,4-dion.
23. 3-(4-Chlorobenzyl)-5,6-diphenyl-tetrahydro-1,3-thiazine-2,4-dion.
24. 3-Benzyl-5,5-diphenyl-tetrahydro-1,3-thiazine-2,4-dion.
25. 3-Benzyl-5,5-di-(4-tolyl)-tetrahydro-1,3-thiazine-2,4-dion.

## Revendications

1. Produit de traitement de la peau renfermant des supports et additifs physiologiquement compatibles caractérisé en ce qu'il renferme au moins une tétrahydro-1,3-thiazine-2,4-dione répondant à la formule générale I :

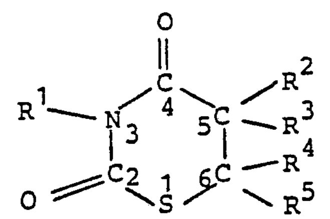

(I)

dans laquelle :

$R^1$ représente l'un des restes suivants : H, alkyle hydroxy-alkyle, carboxy-alkyle, halogéno-alkyle, cyano-alkyl, alcoxy-alkyle, phényle, phényl-alkyle, un phényle ou un phényl-alkyle alkylo-, halogéno-, nitro-, alkoxy-, phényloxy- ou cyano-substitué en 4$^e$ position, pyridyle, thiazolyle, benzothiazolyle, thiényle, furyle, pyrazolyle, imidazolyle, pyridazinyle, thiadiazolyle, pyrimidinyle et thiazinyle,

$R^2$ et $R^3$ représentent, indépendamment l'un de l'autre, H, un halogène, un alkyle, un carboxyle, un phényle, un phényl-alkyle, un phényle alkylo-substitué en 4$^e$ position,

$R^4$ est l'un des restes suivants : H, halogène, alkyle halogéno-alkyle, carboxy-alkyle, phényl-alkyle, phényle, phényle alkylo- substitué en 4$^e$ position, 2-furyle, 2-furyle substitué et

$R^5$ représente H, un halogène, un alkyle, un phényl-alkyle, un phényle ou un phényle alkylo-substitué en 4$^e$ position (où « alkyle » signifie alkyle inférieur) avec la condition que l'un au moins des restes $R^2$, $R^3$, $R^4$ et $R^5$ ne soit pas H lorsque $R^1$ est un phényle halogéno substitué en 4$^e$ position.

2. Produit selon la revendication 1, caractérisé en ce que les composés répondant à la formule I sont présents dans une proportion totale de 0,1 à 5 % en poids, de préférence de 0,5 à 3 % en poids.

3. Produit de traitement de la peau selon les revendications 1 et 2, caractérisé en ce que c'est un produit de protection de la peau.

4. Produit de traitement de la peau selon les revendications 1 et 2, caractérisé en ce que c'est un produit prophylactique de protection contre le soleil (produit anti-solaire).

5. Produit de traitement de la peau selon les revendications 1 et 2, caractérisé en ce que c'est un produit prophylactique de protection contre le froid.

6. Produit de traitement de la peau selon les revendications 1 et 2, caractérisé en ce que c'est un produit prophylactique contre le vieillissement de la peau.

7. Produit selon les revendications 1 à 6, caractérisé en ce qu'il se présente sous la forme d'une crème.

8. Utilisation de composés répondant à la formule générale I dans des produits de traitement de la peau.

9. Composés répondant à la formule générale III :

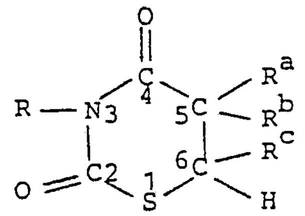

(III)

dans laquelle R représente H, un groupe alkyle, phényle, phényl-alkyle, un phényle ou un phényl-alkyle alkylo-, halogéno-, nitro-, alkoxy-, phényloxy- ou cyano- substitué en 4e position, $R^a$ représente H, un groupe phényle ou un groupe phényle alkylo- substitué en 4e position, $R^b$ représente H, un groupe phényle ou un groupe phényle alkylo- substitué en 4e position, et $R^c$ représente H, un groupe alkyle ou un groupe phényle (alkyle signifiant alkyle inférieur), à condition que $R^a$, $R^b$ et $R^c$ ne représentent simultanément H que lorsque R représente un groupe phényl-alkyle substitué en 4e position et que R ne représente H que lorsque $R^a$ et $R^b$ représentent un groupe phényle et $R^c$ représente H.

10. Utilisation de composés répondant à la formule générale III dans des produits de traitement de la peau.

11. 3-(4-chlorobenzyl)-tétrahydro-1,3-thiazine-2,4-dione.

12. 3-(4-chlorphényl)-6-méthyl-tétrahydro-1,3-thiazine-2,4-dione.

13. 3-benzyl-6-méthyl-tétrahydro-1,3-thiazine-2,4-dione.

14. 6-méthyl-3-(2-phényléthyl)-tétrahydro-1,3-thiazine-2,4-dione.

15. 5,5-diphényl-tétrahydro-1,3-thiazine-2,4-dione.

16. 3-méthyl-5,5-diphényl-tétrahydro-1,3-thiazine-2,4-dione.

17. 6-phényl-3-(4-méthoxyphényl)-tétrahydro-1,3-thiazine-2,4-dione.

18. 6-phényl-3-(2-phényléthyl)-tétrahydro-1,3-thiazine-2,4-dione.

19. 3-éthyl-5,5-diphényl-tétrahydro-1,3-thiazine-2,4-dione.

20. 3-méthyl-5,5-di-(4-tolyl)-tétrahydro-1,3-thiazine-2,4-dione.

21. 3-(4-chlorophényl)-5,5-diphényl-tétrahydro-1,3-thiazine-2,4-dione.

22. 3,5,5-triphényl-tétrahydro-1,3-thiazine-2,4-dione.

23. 3-(4-chlorobenzyl)-5,6-diphényl-tétrahydro-1,3-thiazine-2,4-dione.

24. 3-benzyl-5,5-diphényl-tétrahydro-1,3-thiazine-2,4-dione.

25. 3-benzyl-5,5-di-(4-tolyl)-tétrahydro-1,3-thiazine-2,4-dione.